# EUROPEAN PATENT APPLICATION

(11) **EP 4 717 144 A1**
(43) Date of publication of application: **01.04.2026**
(21) Application number: 24203450.2
(22) Date of filing: 30.09.2024
(51) Int. Cl.: A61B 1/005, A61B 1/008

(54) **ENDOSCOPE HAVING AN INTEGRAL BENDING SECTION BODY WITH FLEXIBLY CONNECTED BENDING SEGMENTS**

(71) Applicant: Ambu A/S, 2750 Ballerup (DK)
(72) Inventor: HANSEN, Frederik Clausager Vemb, 2400 Copenhagen NV (DK)
(74) Representative: Winter, Brandl - Partnerschaft mbB

(57) **Abstract**

The present disclosure relates to an endoscope (2) comprising: an endoscope handle or interface (4); and an insertion cord (6) configured to be inserted into a patient's body cavity and comprising an actively bendable bending section (10), the bending section (10) comprising an integral bending section body (16, 16') made of polymer material, the bending section body (16, 16') comprising a plurality of interconnected bending segments (18, 18'), wherein hinges (26, 26') between adjacent bending segments (18, 18') are formed by hinge protrusions (30, 30') engaging in hinge recesses (32, 32'), and wherein flexible connection portions (34, 34') are provided between the adjacent bending segments (18, 18'), for providing an interconnection between the adjacent bending segments (18, 18').

## Description

The present disclosure relates to an endoscope comprising: an endoscope handle or interface; and an insertion cord configured to be inserted into a patient's body cavity and comprising an actively bendable bending section, the bending section comprising an integral bending section body made of polymer material, the bending section body comprising a plurality of interconnected bending segments.

### Related Art

Endoscopes which include specialized instruments such as bronchoscopes, arthroscopes, colonoscopes, laparoscopes, gastroscopes, duodenoscopes and ureteroscopes are well known from the related art and are used for visual examination and diagnosis of hollow organs and body cavities, as well as to assist in surgery, e.g. for a targeted tissue sampling. Both reusable and disposable (i.e. single-use) endoscopes are known from the related art. Known endoscopes usually comprise an endoscope handle via which an operator can hold and control the endoscope. An insertion cord comprising an insertion tube, an actively bendable bending section, which is a section of increased flexibility, and a distal tip unit is usually connected to the endoscope handle. The insertion cord is configured to be inserted into the hollow organs and body cavities of a patient.

Known endoscopes usually contain steering wires that are pulled and released to bend the flexible bending section of the endoscope, in order to tilt the distal tip unit. In order to achieve a bending of the bending section, a rotating force being applied to e.g. a handle wheel or a lever provided at the proximal endoscope handle by a user may be transmitted into a pulling force acting on the steering wires in an axial direction of the steering wires.

Single-use endoscopes are already known which have a bending section comprising a bending section body molded in one single piece or two single pieces of a polymer material. The bending section body of such a single-use endoscope comprises a number of rigid bending segments kept together, i.e. connected by bendable hinges. Between adjacent bending segments of the bending section body usually two hinge members or hinge parts are provided, which are arranged approximately diametrically opposed with respect to a center axis of the bending section body. The hinge members are formed as foil hinges, i.e. as short strips of bendable bridges of material between adjacent bending segments allowing the material to bend in an elastic manner between adjacent bending segments.

DE 10 2021 113 183 A1 e.g. discloses a single-use endoscope comprising such a bending section. The bending section disclosed in DE 10 2021 113 183 A1 has a varying length of hinges. In particular, the length of the hinges predominantly increases towards a distal end segment of the bending section, in order to obtain a preferred bending shape when the bending section is fully bent. During assembly of the endoscope disclosed in DE 10 2021 113 183 A1 the steering wires are pretensioned, i.e. pulled, resulting in a compression load on the bending section body. Via this pretension provided to the steering wires, a kind of inherent elasticity is taken from the bending section body. This makes it possible that when an operator operates the endoscope, in particular pulls the steering wires, the bending section body is directly bent in the desired direction, i.e. the bending section body does not have to be elastically deformed or compressed first. The bending section body disclosed in DE 10 2021 113 183 A1, in particular a distal portion of the bending section body with longer hinges, is however susceptible to compression loads. In particular, hinges can collapse to some extent, resulting in an undesired S-shaped deformation or snaking of the bending section body. Such an S-shaped deformation leaves an operator of the endoscope with an inferior overall impression of the single-use endoscope and may also affect a bending performance during an endoscopic procedure performed by the operator.

From EP 4 282 317 A1 there is also already known a single-use endoscope having a bending section comprising a plurality of annular or tubular bending segments made from a polymer material. Hinges between the bending segments are formed by hinge protrusions engaging in hinge recesses. The bending segments have a rather complex design in order to make it possible to keep together the plurality of bending segments by steering wires and to provide the bending section with a desired stability. Moreover, assembly of the bending section is rather complex due to the plurality of separate bending segments, resulting in - especially for single-use endoscopes - undesired higher manufacturing and assembly costs.

In reusable endoscopes, usually metal bending sections are applied. E.g., there is known a metal bending section, which comprises a plurality of separate bending segments, which are riveted together. Moreover, a one-part metal bending section, which is laser cut, is known. Such metal bending sections are rather insusceptible to compression loads, which means that high compression loads can be applied on them. Processing and manufacturing costs are however too high to apply metal bending sections on single-use endoscopes.

### Brief description of the disclosure

The tasks and objectives of the present disclosure are to eliminate or at least to reduce the disadvantages of the related art. In particular, an endoscope shall be provided which is designed for single-use, i.e. which has low manufacturing and assembly costs, and which has a bending section that suitably carries compression loads.

The tasks and objectives of the present disclosure are solved by an endoscope in accordance with claim 1 and by a system in accordance with claim 15. Advantageous embodiments are claimed in the dependent claims and/ or are explained below.

In the present disclosure "proximal" basically means "in a direction away from a patient towards a user" and "distal" basically means "in a direction towards the patient away from the user".

The present disclosure relates to an endoscope comprising: an endoscope handle or interface; and an insertion cord configured to be inserted into a patient's body cavity and comprising an actively bendable bending section, the bending section comprising an integral bending section body made of polymer material, the bending section body comprising a plurality of interconnected bending segments, wherein hinges between (two) adjacent bending segments are formed by hinge protrusions engaging in hinge recesses, and wherein flexible connection portions are provided between the (two) adjacent bending segments, for providing an interconnection between the (two) adjacent bending segments.

The endoscope according to the present disclosure is preferably a low-cost, lightweight, single-use endoscope, which is intended to be disposed after use. This means that the endoscope is preferably optimized for one single use. The endoscope preferably has a limited number of elements, which are preferably manufactured with a low-cost material (polymer/ plastic/ resin) in preferably a low-cost manufacturing process (plastic/ injection molding) and which can be easily assembled. Compared to traditional reusable endoscopes, the focus of the present disclosure is to provide an endoscope which is only used once and which thus does not have to withstand rather aggressive cleaning or sterilization processes and general harsh handling over the life cycle of the endoscope.

The endoscope according to the present disclosure is preferably a small-diameter endoscope, in particular a bronchoscope, a ureteroscope or a cholangioscope (used as a baby endoscope in combination with a duodenoscope), etc. However, the present disclosure is neither limited to the endoscope being any one of the mentioned specific small-diameter endoscopes nor limited to the endoscope being a small-diameter endoscope. Said differently, the endoscope according to the present disclosure may advantageously also be another specialized medical instrument like a duodenoscope, bronchoscope, arthroscope, colonoscope, laparoscope, gastroscope, etc.

The endoscope according to the present disclosure is preferably a one-plane bending endoscope configured to bend in two opposite directions (e.g. up-down). Alternatively, the endoscope may be a two-plane bending endoscope configured to bend in four directions (e.g. up-down and left-right).

The endoscope of the present disclosure comprises an endoscope handle or interface. The endoscope handle may be considered as an interface from which the insertion cord extends. The endoscope handle may comprise control actuators, including manual control actuators. Alternatively, an interface may be provided which is connected to the insertion cord and is detachably connected to e.g. a robotic arm. The endoscope handle or interface according to the present disclosure is preferably provided and configured to control a position of the insertion cord.

Preferably, the insertion cord of the endoscope is connected to the endoscope handle or interface and comprises an insertion tube, the actively bendable bending section and a distal tip unit extending in this order in the proximal-distal direction.

The bending section comprises an integral bending section body made of polymer material. The bending section body is thus an integral part being formed as a single piece from, preferably thermoplastic, polymer material. The bending section body is preferably manufactured in an injection-molding process. It is to be understood that the bending section comprises preferably only one such bending section body. However, the present disclosure is not limited to this embodiment, and in an alternative, a plurality of integral bending section bodies connected to each other may be provided.

The bending section body according to the present disclosure comprises the plurality of interconnected bending segments. I.e. the plurality of bending segments are integrally connected with each other, thereby forming in combination the integral bending section body, and are thus not separate or independent parts. In particular, the plurality of interconnected bending segments may comprise a proximal end segment, a plurality of intermediate segments and a distal end segment. The proximal end segment of the bending section body is the proximal-most bending segment of the plurality of interconnected bending segments and is preferably adapted to be connected to the insertion tube of the insertion cord. The distal end segment of the bending section body is the distal-most bending segment of the plurality of interconnected bending segments and is preferably adapted to be connected to the distal tip unit of the insertion cord. Said differently, the proximal end segment and the distal end segment are preferably designed or adapted such that they may be suitably connected to a remainder of the insertion cord and thus provide suitable interface parts fitting to the remainder of the insertion cord.

According to the present disclosure, hinges between two adjacent bending segments are formed by hinge protrusions engaging in hinge recesses. I.e. a hinge is preferably formed by a hinge protrusion or hinge protrusions of a bending segment being received and in contact with a hinge recess or hinge recesses of an adjacent bending segment. The hinge protrusions and hinge recesses may be formed so as to allow a rolling movement or motion in an interface between the hinge protrusions and the hinge recesses, resulting in a desired bending movement of two adjacent bending segments. Hinges are thus preferably not formed as foil hinges, i.e. are not formed as short strips of bendable bridges of material between adjacent bending segments allowing the material to bend in an elastic manner between adjacent bending segments. In other words, two adjacent bending segments are preferably not connected via hinges or hinge members according to the present disclosure, but have an unconnected hinge interface! unconnected hinge interfaces.

Hinges between two adjacent bending segments may include a hinge between the proximal end segment and an adjacent intermediate segment, hinges between adjacent intermediate segments, and a hinge between the distal end segment and an adjacent intermediate segment. Between adjacent bending segments of the bending section body usually two hinge interfaces are provided, i.e. two hinge protrusions engaging in two hinge recesses. The hinge interfaces are preferably arranged approximately diametrically opposed with respect to a center axis of the bending section body. It may be provided that the intermediate segments are identically formed, i.e. each intermediate segment may comprise a hinge protrusion or hinge protrusions on one axial end and may comprise a hinge recess or hinge recesses on the other axial end. Alternatively, different intermediate segments arranged one after the other in an axial direction of the bending section body may be provided. E.g. it may be provided that a first intermediate segment has only hinge protrusions however no hinge recesses on both axial ends and a second intermediate segment has only hinge recesses however no hinge protrusions on both axial ends.

Since according to the present disclosure the hinges are formed by unconnected hinge interfaces, i.e. hinge protrusions engaging in hinge recesses, the bending section body is however nevertheless an integral part made of polymer material and has interconnected bending segments, flexible connection portions are provided between two adjacent bending segments, so that the interconnection between the two adjacent bending segments is provided by the flexible connection portions. The flexible connection portions thus make it possible that the bending section body is the claimed integral part, which is preferably (injection-)molded in one piece, and thus does not have to be assembled from separate or unconnected bending segments.

The bending section body is preferably manufactured such that the hinge protrusions are spaced or distanced from the hinge recesses. During assembly of the endoscope, it is preferably provided that steering wires are pretensioned, i.e. pulled, resulting in a compression load on the bending section body, said compression load leading to an elastic deformation, i.e. some kind of pre-bending, of the flexible connection portions and leading to the hinge protrusions abutting the hinge recesses. In other words, via the pretension provided to the steering wires, a kind of inherent elasticity is taken from the bending section body, leading to the hinge protrusions contacting or touching, and thus engaging the hinge recesses. Preferably, the hinge protrusions are pressed into the hinge recesses until a kind of hysteresis state is reached. This makes it possible that when an operator operates the endoscope, in particular pulls the steering wires, the bending section body is directly bent in the desired direction, i.e. the bending section body does not have to be elastically deformed or compressed first. It is thus preferred that in an assembled state of the endoscope the hinge protrusion and the hinge recess forming a hinge interface abut and thus engage each other.

The bending section body according to the present disclosure may be described as having a compression resistant hinge design. The hinge protrusion may be designated as convex compression limiting part or portion and the hinge recess may be designated as concave compression limiting part or portion. The compression resistant hinge design according to the present disclosure preferably prevents any collapse of hinges during assembly and thus prevents any S-shaped deformation of the bending section body, coming along with preferably no impairment of the bending performance in an endoscopic procedure performed by an operator. The compression limiting parts or portions preferably abut each other after a pre-tensioning of steering wires was performed during assembly of the endoscope. This means that after assembly and during use of the endoscope, there is preferably always contact between the two compression limiting parts. Thereby, it is avoided that the compression limiting parts or portions need to be moved axially to compress and decompress the bending section body during each bending cycle.

The present disclosure makes it possible that the hinge protrusion, which may be designated as the convex compression limiting part or portion, may be structurally optimized to withstand compression forces. E.g., the hinge protrusion may be a short and wide protrusion suitable to carry compression forces. On the other hand the flexible connection portions may be structurally optimized to provide a desired, especially high, flexibility. E.g., the flexible connection portions may be long and thin material bridges between two adjacent bending segments. According to the present disclosure, it is thus possible to optimize the two functions "Compression Resistance" and "Flexibility" independently since the two functions are split into two separate features. In particular, the "Compression Resistance" can be optimized by suitably designing the hinge interface, in particular the hinge protrusion and the hinge recess, and the "Flexibility" can be optimized by suitably designing the flexible connection portions.

A major advantage of the bending section body according to the present disclosure is that both functions "Compression Resistance" and "Flexibility" can be optimized independently without having to increase the thickness, i.e. the extension in radial direction, of the bending section body. E.g., the flexibility of the flexible connection portions may be suitably adjusted by selecting a suitable length in axial direction and a suitable width in circumferential direction, and the compression resistance may be suitably adjusted by selecting a suitable, rather short, length in axial direction and a suitable, rather big, width in the circumferential direction. Therefore, a ratio between inner diameter ID and outer diameter OD of the bending section body can be optimized. Note that it is desirable in endoscopic procedures to have an outer diameter OD of the bending section body which is as small or thin as possible, in order to be able to insert the insertion cord in a respective body cavity, and to have an inner diameter ID which is as big as possible, in order to be able to provide a working channel tube inside the bending section body having an inner diameter which is as big or large as possible.

In traditional single-use endoscopes, having e.g. a bending section as disclosed in DE 10 2021 113 183 A1, with respect to the foil hinges, which are short strips of bendable bridges of material between adjacent bending segments, usually a compromise needs to be found as follows: On the one hand, the hinges should be thick enough to withstand compression forces and thus to avoid the S-shaped deformation, which may also be referred to as "snaking", of the bending section. On the other hand, the hinges should be as thin as possible to reduce stress in the polymer material and to ensure low forces during bending.

Preferably, the hinge between two adjacent bending segments comprises two diametrically opposed hinge interfaces, each hinge interface being formed by a hinge protrusion engaging in a hinge recess. In other words, preferably two hinge protrusions engage in two hinge recesses, in particular a first hinge protrusion engages in a first hinge recess (first hinge interface) and a second hinge protrusion engages in a second hinge recess (second hinge interface), between two adjacent bending segments.

According to a preferred embodiment, each hinge interface being formed by a hinge protrusion engaging in a hinge recess is encircled or surrounded by two flexible connection portions. In other words, there may be provided a first flexible connection portion clockwise in circumferential direction next to the hinge interface and a second flexible connection portion anticlockwise in circumferential direction next to the hinge interface. Preferably, the flexible connection portions encircling or surrounding the hinge interface are arranged directly next to or adjacent to the hinge interface. An arrangement directly next to or adjacent to the hinge interface means that the flexible connection portions are spaced by less than 90° in circumferential direction, preferably by less than 45° in circumferential direction, from the hinge interface, in particular from a center of the hinge interface. Each flexible connection portion is preferably connected to both adjacent bending segments. In case of the provision of two diametrically opposed hinge interfaces, there are thus - according to this preferred embodiment - preferably provided four flexible connection portions, wherein two flexible connection portions surround or encircle the first hinge interface formed by the first hinge protrusion engaging in the first hinge recess and two flexible connection portions surround or encircle the second hinge interface formed by the second hinge protrusion engaging in the second hinge recess.

The flexible connection portions may be curved or angled portions having a bend point or inflexion point. The bend point or inflexion point may be provided halfway in between the two adjacent bending segments. In particular, it may be provided that the flexible connection portions extend from one bending segment of the two adjacent bending segments, both in axial direction and in circumferential direction away from the hinge interface, towards the bend point or inflexion point, from which the flexible connection portions extend, both in axial direction and in circumferential direction towards the hinge interface, to the other bending segment of the two adjacent bending segments.

Two flexible connection portions surrounding or encircling a hinge interface may be symmetrically identical with respect to a bending plane defined by the hinge interface, in particular by the two diametrically opposed hinge interfaces.

It may be provided that the flexible connection portions elastically deform when the steering wires are pretensioned, i.e. pulled during assembly of the endoscope. In particular, the flexible connection portions are provided and configured to deform elastically when the hinge protrusions approach and abut the hinge recesses during pretensioning of the steering wires. E.g., a portion of the flexible connection portion upstream of the bend point or inflexion point may approach a portion of the flexible connection portion downstream of the bend point or inflexion point, the portions thus axially approaching each other while being bent around the bend point or inflexion point.

The preferred design of two flexible connection portions surrounding or encircling a hinge interface, and being especially preferred symmetrically identical, has the following advantages: In the assembled state of the endoscope, when the operator performs a bending movement of the bending section, one of the two flexible connection portions will be further bent, i.e. compressed, while the other flexible connection portion will be stretched. The two flexible portions surrounding or encircling the hinge interface advantageously exert a force on the two adjacent bending segments, which will bring the bending section back to a neutral non-bent position when there is no pull on one of the steering wires in addition to the factory pre-tensioning. The flexible connection portions according to an especially preferred aspect of the present disclosure may thus be configured and provided to apply a force to the bending section body which brings the bending section body back to a neutral position, in particular to urge or force the bending section body back to the neutral position when there is no pull on one of the steering wires.

According to another alternative embodiment, the flexible connection portions do not surround or encircle the hinge interface formed by the hinge protrusion engaging the hinge recess but are spaced from the hinge interface. E.g., two flexible connection portions may be provided. Each flexible connection portion may especially preferred be spaced by around 90° in the circumferential direction from the hinge interface(s).

It may be provided that the flexible connection portions providing the interconnection between two adjacent bending segments do not serve to urge or force the bending section body back into a neutral, non-bent position when there is - compared to the factory pretensioning - no additional pull on one of the steering wires. In particular, the flexible connection portions according to an embodiment may serve another function. As mentioned above, it may be provided that the flexible connection portions elastically deform when the steering wires are pretensioned, i.e. pulled during assembly of the endoscope. In particular, the flexible connection portions may be provided and configured to deform elastically when the hinge protrusions approach and abut the hinge recesses during pretensioning of the steering wires. According to an embodiment, the flexible connection portions may be designed and configured to provide guiding apertures for steering wires in the assembled state of the endoscope, i.e. in a state in which the hinge protrusions abut the hinge recesses.

According to the alternative embodiment, each flexible connection portion may comprise two apertures and a predetermined bend or kink point or line or portion arranged in the axial direction between the two guiding apertures. There may be additional kink points or lines on both sides of the apertures to control the behavior under compression. In other words, in the axial direction, a first aperture of the two apertures may be closer to one bending segment of the two adjacent bending segments, and a second aperture of the two apertures may be closer to the other bending segment of the two adjacent bending segments, with the predetermined bend or kink point or line or portion arranged axially in between the two apertures. In still other words, each flexible connection portion may be described as comprising two longitudinal or axial webs essentially arranged in parallel with respect to each other, with a cross web forming the predetermined bend or kink point or line or portion and connecting the two longitudinal or axial webs, such that the two apertures are formed.

According to the alternative embodiment, when the steering wires are pretensioned, i.e. pulled during assembly of the endoscope, the hinge protrusion(s) abut the hinge recesse(s) and the flexible connection portions bend or kink radially inwards at the predetermined bend or kink point or line or portion, wherein via the inward bending or kinking of the flexible connection portions, the guiding apertures for the steering wires are formed. In a variation, the flexible connection portions could bend outward instead of inwards. This would have the effect that the working channel is not being squeezed inside the bending section during bending. In this variation the flexible connection may preferably be designed to have rounded shapes in order to not affect the tissue when moved in body cavities. The bending cover provided on the external side of the bending section would also limit any interference with the tissue.

Preferably, the apertures are oval, i.e. have an oval shape, thereby enabling the steering wires to move freely, in particular independently of an actual bending angle of the bending section. In particular, it has turned out that in order to obtain round guiding apertures in a top view onto the bending section body, the apertures provided in the flexible connection portions are preferably oval. I.e. by bending or kinking the flexible connection portions radially inwards at the predetermined bend or kink point or line or portion, the oval apertures form essentially round or circular guiding apertures, seen in the top view. The apertures could also be elongated slots.

The bending section body may comprises one large inner lumen for accommodating a working channel tube, potentially also for further tubes or electrical cables. Alternatively, there may be one large inner lumen, especially for the working channel tube, and another inner lumen, e.g. for electrical cables.

There may be provided two steering wire lumen, namely a first steering wire lumen and a second steering wire lumen, each steering wire lumen being preferably a rather small lumen and configured and provided to accommodate a first steering wire respectively a second steering wire. The steering wire lumen may be provided in addition to the one or more above-mentioned inner lumen. The steering wire lumen may be spaced by around 90° in circumferential direction from the diametrically opposed hinge interfaces. Note that according to the alternative embodiment a provision of a steering wire lumen is not necessary (due to the provision of the guiding apertures for the steering wires). Therefore, according to the alternative embodiment, preferably no additional steering wire lumen are provided. Thereby, the need for long thin molding cores, in the molding process of the bending section body may be avoided. This may reduce the manufacturing costs. This may also enable a larger ratio between the inner diameter and the outer diameter of the bending section body.

According to a preferred embodiment a polymer material of the bending section body is a rather hard and stiff polymer material, in particular a less flexible polymer material. In particular, the polymer material of the bending section body may be optimized to provide a suitable torsional stiffness of the bending section body. Especially, the suggested design according to the present disclosure may have some minor drawbacks with respect to torsional stiffness. The design may reduce the torsional stiffness of the bending section body. However, it turned out that torsional stiffness may be regained by manufacturing, in particular molding, the bending section body with a harder and stiffer material, e.g. polypropylene.

The present disclosure also relates to a system comprising an endoscope as defined above and a monitor connected to the endoscope.

### Brief description of the figures

The disclosure is explained in more detail below using preferred embodiments and referring to the accompanying figures.
- Fig. 1: is a perspective view showing an endoscope according to the present disclosure;
- Fig. 2: is a perspective view of a bending section of the endoscope;
- Fig. 3: is a cross-sectional view through the bending section of Fig. 2;
- Fig. 4: is a detailed side view of a portion of the bending section of Fig. 2;
- Fig. 5A: a perspective view of a bending section body of the bending section of Fig. 2 in a non-assembled state of the endoscope;
- Fig. 5B: a detailed side view of hinges and flexible connections in a non-assembled state of the bending section, similar to Fig. 4.
- Fig. 6A: a perspective view of the bending section body of the bending section of Fig. 2 in an assembled state of the endoscope;
- Fig. 6B: a detailed side view of hinges and flexible connections in an assembled state of the bending section.
- Fig. 7: is a perspective view of a bending section body according to an alternative embodiment of the present disclosure;
- Fig. 8: is a detailed perspective view of a flexible connection portion of the bending section body of Fig. 7;
- Fig. 9A: is a variation of the flexible connection design in the alternative embodiment, shown in a non-assembled state of the bending section.
- Fig. 9B: is a close up of the flexible connection in Fig. 9A.
- Fig. 10: a cross-sectional view of the bending section of Fig. 9A but shown in an assembled state of the bending section.

The figures are schematic in nature and serve only to understand the disclosure. Identical elements are marked with the same reference signs. The features of the different embodiments can be interchanged among each other.

### Detailed description of preferred embodiments

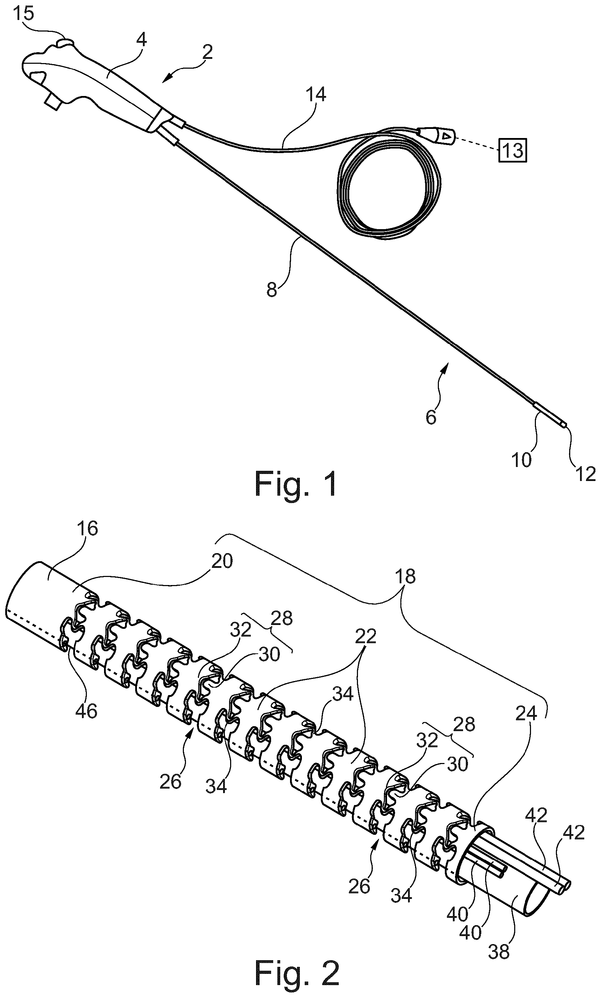

In Fig. 1, an endoscope 2 is shown. The endoscope 2 is preferably a single-use endoscope. The endoscope 2 is preferably, but not necessarily, a small-diameter endoscope such as an ureteroscope. The endoscope 2 comprises a proximal endoscope handle 4 designed to be held by a user/ physician and being configured to accommodate operating parts of the endoscope 2. Further, the endoscope 2 comprises an insertion cord 6, which is configured to be inserted into a patient's body cavity. The insertion cord 6 comprises an insertion tube 8, an actively bendable bending section 10 and a distal tip unit 12, extending in this order from the proximal endoscope handle 4.

At the distal tip unit 12, image capturing means such as a miniature video camera and illuminating means such as light-emitting diodes or optical fibers connected to a proximal source of light are arranged/installed, such that the patient's body cavity can be illuminated and inspected. An image captured by the image capturing means can be shown on a monitor 13. The monitor 13 is provided separately from and connected/ connectable with the endoscope 2 via a cable 14.

The endoscope handle 4 comprises an operating unit 15, which is preferably formed as a lever, for steering the bending section 10 of the insertion cord 6. In particular, a rotation/ turning force can be applied to the operating unit 15 by the user. The distal tip unit 12 may be tilted/ moved by bending the bending section 10. In particular, the operating unit 15 can be operated by the user to tilt the distal tip unit 12 in a bending plane (e.g. up-down). The bending section 10 may be largely covered by a flexible cover for preventing contamination.

The endoscope 2 may comprise steering wires (not shown in Fig. 1) for controlling the bending movement of the bending section 10. The steering wires may be connected to the operating unit 15. The steering wires may extend through the insertion tube 8 and the bending section 10. By turning the operating unit 15, steering wires/ steering wire portions can be pulled and released and the distal tip unit 12 can tilt according to a direction in which the operating unit 15 is rotated. In other words, by operating the operating unit 15 the user is able to tilt the distal tip unit 12 in the bending plane by bending the bending section 10 correspondingly.

It is to be understood that although Fig. 1 shows a one-plane bending endoscope, the present disclosure is not limited to the endoscope 2 being a one-plane bending endoscope. I.e. the endoscope 2 may also be a two-plane bending endoscope configured for bending in a first bending plane and a second bending plane, wherein the second bending plane is preferably perpendicular to the first bending plane.

Fig. 2 shows a perspective view of the bending section 10 with the flexible cover removed. The bending section 10 comprises a bending section body 16, which is an integral piece of polymer material, preferably manufactured by injection molding a thermoplastic polymer material, especially preferred a rather hard and stiff polymer material like polypropylene or polyoxymethylene (POM). The bending section body 16 comprises a plurality of interconnected bending segments 18 including a proximal end segment 20, a plurality of intermediate segments 22 and a distal end segment 24. The proximal end segment 20 is connected to the insertion tube 8. The distal end segment 24 is connected to the distal tip unit 12. A hinge 26 between adjacent bending segments 18 is formed by two diametrically opposed hinge interfaces 28, wherein each hinge interface 28 comprises a hinge protrusion 30 and a hinge recess 32. Flexible connection portions 34 provide an interconnection between adjacent bending segments 18.

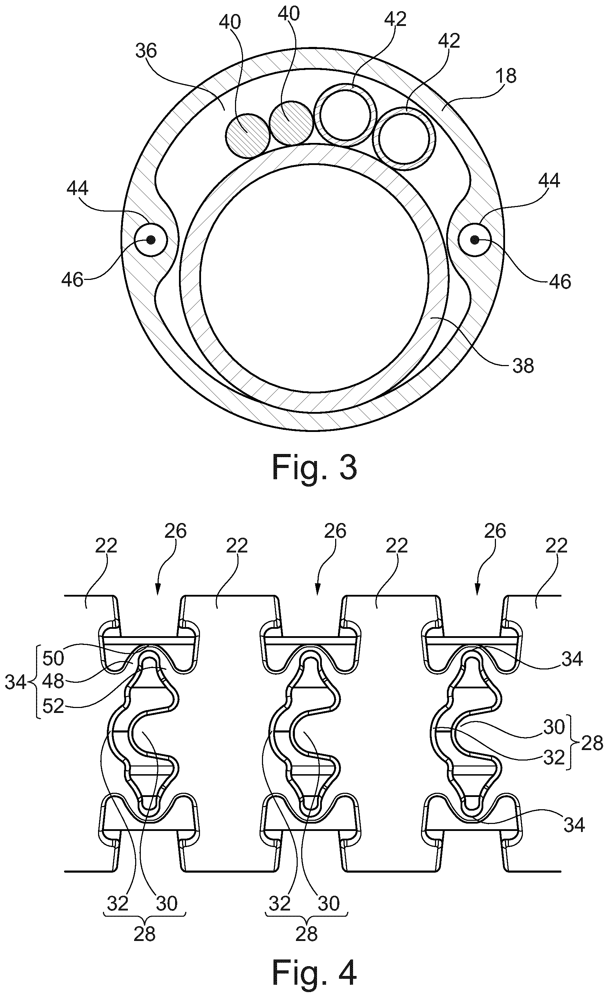

With reference also to Fig. 3, which is a cross-sectional view through the bending section 10, in particular through a bending segment 18 of the bending section 10, in addition to Fig. 2, it is apparent that the bending section body 16 comprises or forms one large inner lumen 36, in which a large working channel tube 38, electrical cables 40, and potentially further tubes 42 like a rinsing tube or an insufflation tube are accommodated. The bending section body 16 further comprises or forms two small steering wire lumen 44, in which steering wires 46 are accommodated and guided through. The steering wire lumen 44 are arranged diametrically opposed with respect to each other and are spaced by around 90° in a circumferential direction from the hinge interfaces 28.

With reference also to Fig. 4, which is a detailed side view of a portion of the bending section 10 shown in Fig. 2, in addition to Fig. 2, a state during assembly of the endoscope 2 is shown, in which the working channel tube 38, the electrical cables 40 and the tubes 42 are already arranged in and guided through the large inner lumen 36, and in which the steering wires 46 are already arranged in and guided through the steering wire lumen 44. However, the steering wires 46 are not yet fixed to the distal end segment 24 and the steering wires 46 thus also have not yet been pretensioned, i.e. pulled.

In the state during assembly shown in Figs. 2 and 4 the hinge protrusions 30 are spaced from the hinge recesses 32, i.e. the hinge protrusions 30 do not yet engage in the hinge recesses 32 and are thus not in contact with them, respectively do not abut them. Each hinge interface 28 formed by the hinge recess 32 and the associated hinge protrusion 30, which is provided to be engaged with the respective hinge recess 32 in the assembled state of the endoscope 2, is encircled or surrounded by two flexible connection portions 34. One flexible connection portion 34 is arranged directly next to, i.e. adjacent the hinge interface 28 clockwise in circumferential direction and one flexible connection portion 34 is arranged directly next to, i.e. adjacent the hinge interface 28 anticlockwise in circumferential direction. Both flexible connection portions 34 are spaced by less than 45° in circumferential direction from a center of the hinge interface 28. Each flexible connection portion 34 is connected to both adjacent bending segments 22. In particular, an upstream portion 48 of each flexible connection portion 34 extends from one bending segment 22 of two adjacent bending segments 22, both in axial direction and in circumferential direction away from the hinge interface 28, towards a bend point or inflexion point 50, from which a downstream portion 52 extends, both in axial direction and in circumferential direction towards the hinge interface 28, to the other bending segment 22 of the two adjacent bending segments 22. The flexible connection portions 34 are thus curved or angled and have the bend point or inflexion point 50 in axial direction halfway in between the two adjacent bending segments 22. The two flexible connection portions 34 surrounding or encircling the hinge interface 28 are symmetrically identical with respect to a bending plane defined by two diametrically opposed hinge interfaces 28 of the hinge 26. Since each hinge 26 comprises two diametrically opposed hinge interfaces 28, there are provided four flexible connection portions 34 for each hinge 26.

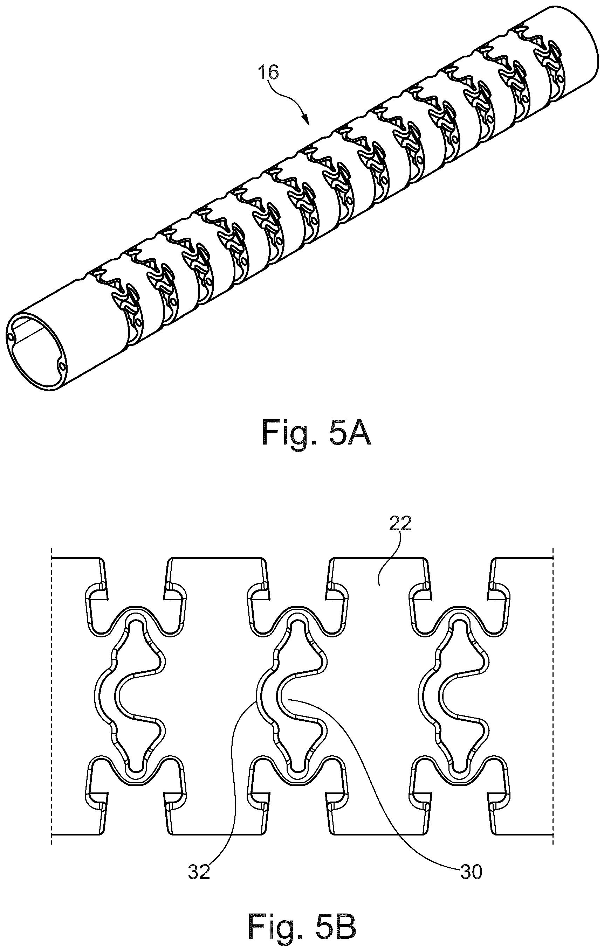

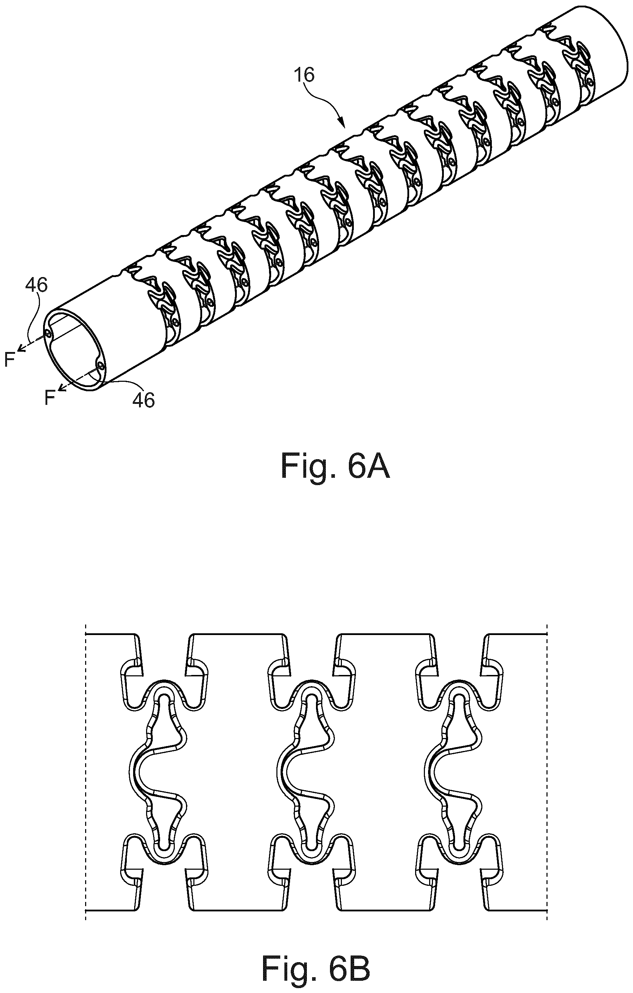

Figs. 5A and 5B show a perspective view and a detailed side view of the bending section body 16 in the same state as in Fig. 2 and Fig. 4, i.e. in a state in which the hinge protrusions 30 are spaced from the hinge recesses. Figs. 6A and 6B show a perspective view and a detailed side view of the bending section body 16 in an assembled state of the endoscope 2. For illustration purposes, all elements of the endoscope 2 except for the bending section body 16 and the steering wires 46 were removed in Fig. 6A. During assembly of the endoscope 2, the steering wires 46 are pretensioned, i.e. pulled, cf. force F in Fig. 6A, resulting in a compression load on the bending section body 16. The compression load leads to an elastic deformation of the flexible connection portions 34, in particular to the upstream portion 48 and the downstream portion 52 approaching each other and bending around the bend point or inflexion point 50, and leads also to the hinge protrusions 30 abutting or contacting or touching or engaging the hinge recesses 32. Figs. 6A and 6B show the assembled state of the endoscope 2, in which the hinge protrusions 30 engage in the hinge recesses 32 and the flexible connection portions 34 have already been elastically deformed. When an operator pulls the steering wires 46 in the state shown in Figs. 6A and 6B, the bending section 10 is directly bent in the desired direction (e.g. up or down), since the bending section body16 does not have to be compressed first. In particular, the hinge protrusions 30 and the hinge recesses are formed so as to allow a rolling movement or motion in the hinge interfaces 28 between the hinge protrusions 30 and the hinge recesses 32. One of the flexible connection portions 34 surrounding the hinge interface 28 will be compressed, while the other one of the flexible connection portions 34 surrounding the hinge interface 28 will be stretched. When the operator stops pulling the steering wires 46 the flexible connection portions 34 bring the bending section body 16 back to a neutral, non-bent position due to their inherent material elasticity/ flexibility.

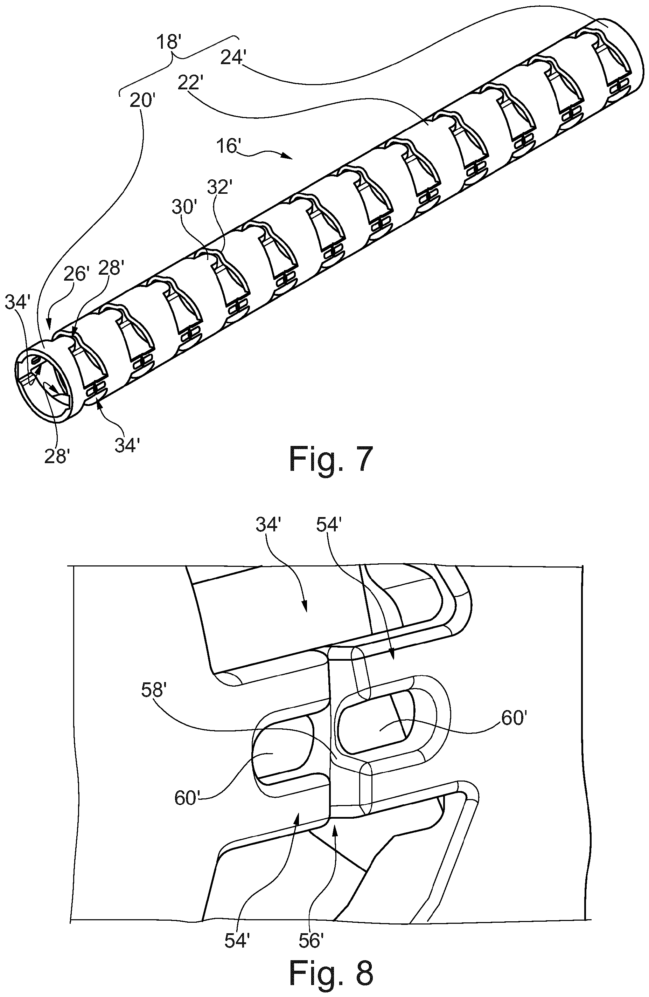

Fig. 7 shows a perspective view of a bending section body 16' according to an alternative embodiment of the present disclosure. The bending section body 16' is an integral piece of polymer material, preferably manufactured by injection molding a thermoplastic polymer material. The polymer may be a rather hard and stiff polymer material like polypropylene, or a more flexible polymer material like POM. The choice of polymer material may depend on the specific design of the flexible connection portions. The bending section body 16' comprises a plurality of interconnected bending segments 18' including a proximal end segment 20', a plurality of intermediate segments 22' and a distal end segment 24'. The proximal end segment 20' is connected to the insertion tube 8. The distal end segment 24' is connected to the distal tip unit 12. A hinge 26' between adjacent bending segments 18' is formed by two diametrically opposed hinge interfaces 28', wherein each hinge interface 28' comprises a hinge protrusion 30' and a hinge recess 32'. Two flexible connection portions 34' provide an interconnection between adjacent bending segments 18'.

The two flexible connection portions 34' according to the alternative embodiment do not surround or encircle the hinge interface 28' formed by the hinge protrusion 30' engaging the hinge recess 32', but are spaced from the hinge interface 28'. The two flexible connection portions 34' are arranged diametrically opposed with respect to each other and are spaced by around 90° in the circumferential direction from the hinge interfaces 28'. With reference also to Fig. 8, which shows a detailed perspective view of one flexible connection portion 34', each flexible connection portion 34' comprises two longitudinal or axial webs 54' arranged essentially in parallel with respect to each other, with a cross web 56' connecting the two longitudinal or axial webs 54' and essentially forming or providing a predetermined bend or kink line 58'. Two oval apertures 60' are formed by the two longitudinal axial webs 54' and the cross web 56', wherein the two apertures 60' are arranged next to, i.e. adjacent each other in the axial direction of the bending section body 16'.

When the steering wires 46 are pretensioned, i.e. pulled during assembly of the endoscope 2 the hinge protrusions 30' engage, i.e. abut, the hinge recesses 32' and the two flexible connection portions 34' deform elastically. In particular, the two flexible connection portions 34' bend or kink radially inwards at the predetermined bend or kink line 58' of the cross web 56'. Via the inward bending or kinking of the flexible connection portions 34' the two oval apertures 60' form and provide guiding apertures for the steering wires 46.

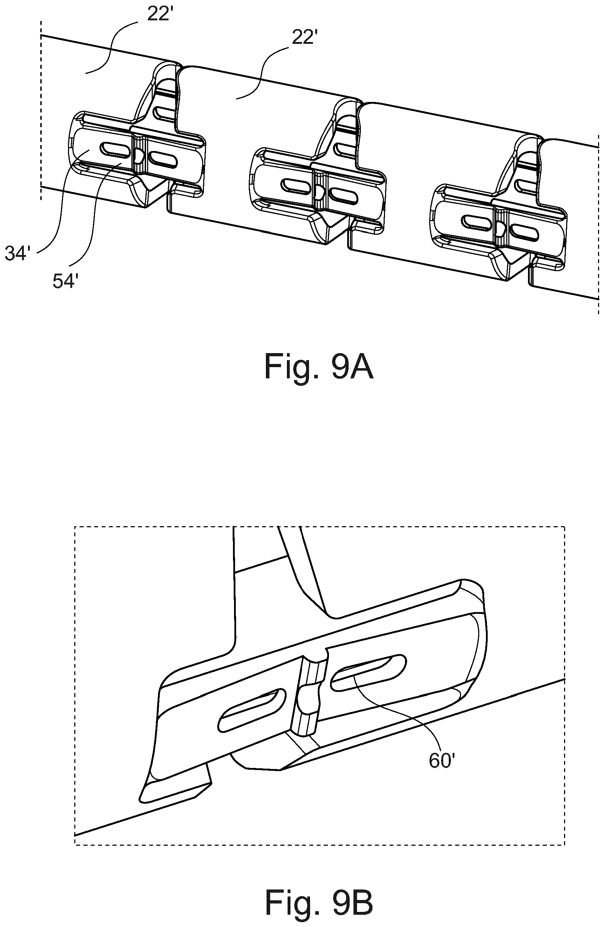

Figs. 9A and 9B show a variation of the flexible connection design in the alternative embodiment. This is shown in a non-assembled state of the bending section. In this variation the flexible connection portion 34' comprises one longitudinal or axial web 54' connecting two neighboring segments 22'. This web 54' is made as a thin film-like layer, which will bend when compressed. One advantage of this design is, that it may be simpler and faster to mold, when the bend or kink line shown in Fig. 8, is not included.

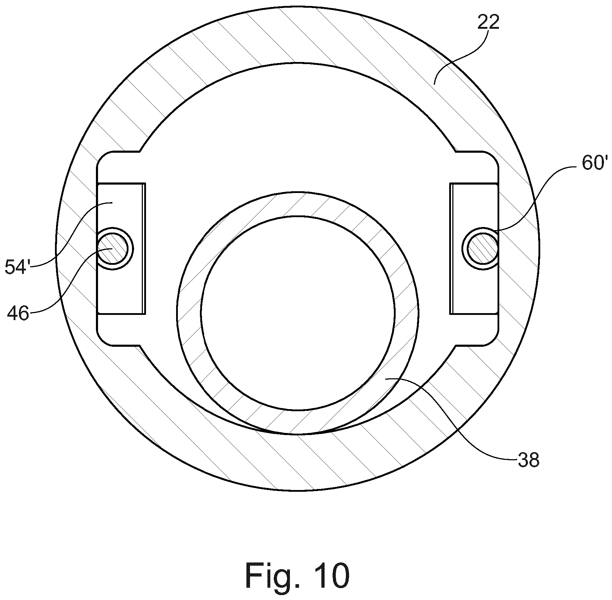

Fig. 10 shows a cross-sectional view of the bending section according to the variation of the alternative embodiment shown in Fig. 9A and 9B but shown in an assembled compressed state of the bending section. The compressed bended webs 54' are seen with the steering wires 46 passing through the holes formed by the apertures 60'. The working channel tube 38 is also shown in Fig. 10.

### List of reference signs

- 2: endoscope
- 4: endoscope handle
- 6: insertion cord
- 8: insertion tube
- 10: bending section
- 12: distal tip unit
- 13: monitor
- 14: cable
- 15: operating unit
- 16, 16': bending section body
- 18, 18': bending segment
- 20, 20': proximal end segment
- 22, 22': intermediate segment
- 24, 24': distal end segment
- 26, 26': hinge
- 28, 28': hinge interface
- 30, 30': hinge protrusion
- 32, 32': hinge recess
- 34, 34': flexible connection portion
- 36: inner lumen
- 38: working channel tube
- 40: electrical cable
- 42: tube
- 44: steering wire lumen
- 46: steering wire
- 48: upstream portion
- 50: bend/ inflexion point
- 52: downstream portion
- 54': longitudinal or axial web
- 56': cross web
- 58': predetermined bend or kink line
- 60': aperture

## Claims

1. Endoscope (2) comprising:
an endoscope handle or interface (4); and
an insertion cord (6) configured to be inserted into a patient's body cavity and comprising an actively bendable bending section (10),
the bending section (10) comprising an integral bending section body (16, 16') made of polymer material,
the bending section body (16, 16') comprising a plurality of interconnected bending segments (18, 18'), wherein
hinges (26, 26') between adjacent bending segments (18, 18') are formed by hinge protrusions (30, 30') engaging in hinge recesses (32, 32'), and wherein
flexible connection portions (34, 34') are provided between the adjacent bending segments (18, 18'), for providing an interconnection between the adjacent bending segments (18, 18').

2. Endoscope (2) according to claim 1, wherein a hinge (26, 26') between two adjacent bending segments (18, 18') comprises two, preferably diametrically opposed, hinge interfaces (28, 28'), wherein each hinge interface (28, 28') is formed by a hinge protrusion (30, 30') engaging in a hinge recess (32, 32').

3. Endoscope (2) according to claim 2, wherein each hinge interface (28) is encircled or surrounded by two flexible connection portions (34), one flexible connection portion (34) being arranged clockwise in circumferential direction next to the hinge interface (28) and one flexible connection portion (34) being arranged anticlockwise in circumferential direction next to the hinge interface (28).

4. Endoscope (2) according to claim 3, wherein the flexible connection portions (34) are spaced by less than 90° in circumferential direction, preferably by less than 45° in circumferential direction, from the hinge interface (28), in particular from a center of the hinge interface (28).

5. Endoscope (2) according to claim 3 or 4, wherein the two flexible connection portions (34) encircling or surrounding the hinge interface (28) are symmetrically identical with respect to a bending plane defined by the two hinge interfaces (28) of the hinge (26).

6. Endoscope (2) according to any one of claim 3 to 5, wherein the flexible connection portions (34) are curved or angled portions extending between two adjacent bending segments (18) and having a bend or inflexion point (50).

7. Endoscope (2) according to any one of claims 3 to 6, wherein the flexible connection portions (34) are configured and provided to apply a force to the bending section body (16) which brings the bending section body (16) back to a neutral, unbent position.

8. Endoscope (2) according to any one of claims 3 to 7, wherein the bending section body (16) comprises at least one inner lumen (36) and at least two steering wire lumen (44).

9. Endoscope (2) according to claim 1 or 2, wherein two diametrically opposed flexible connection portions (34') are provided between two adjacent bending segments (18'), each flexible connection portion (34') being spaced by 90° in a circumferential direction from the hinge interfaces (28').

10. Endoscope (2) according to claim 9, wherein the flexible connection portions (34') are designed and configured to provide guiding apertures (60') for steering wires (46) in an assembled state of the endoscope (2), i.e. in a state in which the hinge protrusions (30') engage the hinge recesses (32').

11. Endoscope (2) according to claim 9 or 10, wherein the guiding apertures (60') have an oval shape or an elongated shape.

12. Endoscope (2) according to any one of claims 1 to 11, wherein the hinge protrusions (30, 30') engage the hinge recesses (32, 32') only in an assembled state of the endoscope (2).

13. Endoscope (2) according to any one of claim 1 to 12, wherein during assembly of the endoscope (2) the bending section body (16, 16') is compressed via a pretensioning of steering wires (46), wherein the hinge protrusions (30, 30') and the hinge recesses (32, 32') are configured and provided to serve as compression limiting parts or portions, and wherein the flexible connection portions (34, 34') are provided and configured to deform elastically.

14. Endoscope (2) according to any one of claims 1 to 13, wherein the bending section body (16, 16') is manufactured in an injection-molding process.

15. System comprising an endoscope (2) according to any one of claims 1 to 14 and a monitor (13) connected to the endoscope (2).
